# EUROPÄISCHE PATENTSCHRIFT

(11) **EP 2 272 547 B1**
(45) Veröffentlichungstag und Bekanntmachung des Hinweises auf die Patenterteilung: **11.01.2017**
(21) Anmeldenummer: 10157347.5
(22) Anmeldetag: 23.03.2010
(51) Int. Cl.: A61L 31/02, A61L 31/14, C23C 24/04, A61L 31/08, C23C 26/00

(54) **Implantat und Verfahren zur Herstellung desselben**
Implant and method for manufacturing same
Appareil de formation d'images et procédé de commande de sa faible puissance

(30) Priorität: 23.06.2009 US 219401 P
(43) Veröffentlichungstag der Anmeldung: 12.01.2011
(73) Patentinhaber: Biotronik VI Patent AG, 6341 Baar (CH)
(72) Erfinder: Bayer, Ullrich, 18211, Admannshagen-Bargeshagen (DE); Klocke, Björn, 8006, Zürich (CH)
(74) Vertreter: Randoll, Sören

(56) Entgegenhaltungen:
- EP-A1- 1 402 849
- EP-A2- 2 206 526
- WO-A1-2008/034066
- WO-A2-2009/050251
- FR-A1- 2 775 919
- US-A1- 2009 118 815
- PEUSTER M ET AL: "Long-term biocompatibility of a corrodible peripheral iron stent in the porcine descending aorta" BIOMATERIALS, ELSEVIER SCIENCE PUBLISHERS BV., BARKING, GB LNKD- DOI:10.1016/J.BIOMATERIALS.2006.05.029, Bd. 27, Nr. 28, 1. Oktober 2006 (2006-10-01), Seiten 4955-4962, XP025097462 ISSN: 0142-9612 [gefunden am 2006-10-01]

## Beschreibung

Die vorliegende Erfindung betrifft ein Verfahren zur Herstellung eines Implantats, insbesondere einer intraluminalen Endoprothese, mit einem Körper enthaltend metallisches Material, vorzugsweise Eisen, insbesondere eine Eisenlegierung, sowie ein entsprechendes Implantat erhältlich oder erhalten durch ein derartiges Verfahren.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen einen Körper in der Form eines ggf. durchbrochenen rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatz zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium und Eisen. Die vorliegende Erfindung bezieht sich vorzugsweise auf Implantate, deren biodegradierbarer Werkstoff zumindest teilweise ein Metall enthält, vorzugsweise Eisen, Mangan, Zink und/oder Wolfram, insbesondere eine Eisenbasislegierung (im Folgenden kurz: Eisenlegierung).

Bei der Realisierung biodegradierbarer Implantate wird angestrebt, die Degradierbarkeit entsprechend der angestrebten Therapie bzw. der Anwendung des jeweiligen Implantats (koronar, intracranial, renal etc.) zu steuern. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Implantate mit einer Eisenlegierung, insbesondere eisenhaltige Stents, sind besonders kostengünstig und einfach herstellbar. Beispielsweise für die Behandlung von Stenosen verlieren diese Implantate allerdings ihre mechanische Integrität bzw. Stützwirkung erst nach einem vergleichsweise langen Zeitraum, d.h. erst nach einer Verweildauer in dem behandelten Organismus von ca. 2 Jahren. Dies bedeutet, dass sich der Kollapsdruck bei eisenhaltigen Implantaten für diese Anwendung über die Zeit zu langsam verringert. Für andere Anwendungen der eisenhaltigen Implantate, beispielsweise in der Orthopädie, ist der Degradationszeitraum jedoch zu kurz.

Im Stand der Technik sind bereits unterschiedliche Mechanismen der Degradationskontrolle von Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material beruhen. Diese führen dazu, dass die Degradationszeit verlängert wird. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Andere Lösungen basieren auf Legierungsbestandteilen des biodegradierbaren Materials des Implantat-Körpers, welche den Korrosionsprozess durch Verschiebung der Lage in der elektrochemischen Spannungsreihe beeinflussen. Weitere Lösungen auf dem Gebiet der gesteuerten Degradation rufen durch das Aufbringen von physikalischen (z.B. lokale Querschnittsveränderungen) und/oder chemische Veränderungen der Stent-Oberfläche (z.B. lokal chemisch unterschiedlich zusammengesetzte Multilayer) Sollbrucheffekte hervor. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende bzw. eine zu große Variabilität der Degradation des Implantats.

In US 2009/0118815 und EP 1 402 849 werden metallische Stents offenbart.

In WO 2008/034066 werden Implantate offenbart.

Ein weiteres Problem im Zusammenhang mit Beschichtungen ergibt sich auch daraus, dass Stents oder andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert bzw. (bei Verwendung einer Formgedächtnislegierung als Implantatmaterial) beispielsweise durch Erwärmung über eine Sprungtemperatur in den expandierten Zustand überführt. Auf Grund dieser Durchmesseränderung ist der Körper des Implantats hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den genannten Beschichtungen ergibt sich somit der Nachteil, dass die Beschichtung während der Verformung des Implantats reißt (z.B. aufgrund der Bildung von Mikrorissen) oder auch teilweise entfernt wird. Hierdurch kann eine unspezifizierte lokalen Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Folglich besteht die Aufgabe der vorliegenden Erfindung darin, ein Verfahren zur Herstellung eines Implantats, das insbesondere metallisches Material aufweist, anzugeben, welches eine Degradation des Implantats im gewünschten Zielkorridor, insbesondere in einem kürzeren Zeitraum, bewirkt. Dabei soll die Degradation zu einem kontrollierbaren Zeitpunkt stattfinden und zusätzlich die Dilatation bzw. Verformung des Implantats ohne nennenswerten Einfluss auf das Degradationsverhalten ermöglichen. Entsprechend besteht die Aufgabe der Erfindung außerdem darin, ein derartiges Implantat zu schaffen.

Die obige Aufgabenstellung wird gelöst durch ein Verfahren umfassend die folgenden Schritte:
a) Bereitstellen des Körpers des Implantats,
b) tribochemische Behandlung mindestens eines Teils der Körperoberfläche mittels Strahlteilchen,
   dadurch gekennzeichnet, dass die tribochemische Behandlung in mindestens zwei Schritten erfolgt, nämlich in einem ersten Schritt eine tribochemische Behandlung mittels Strahlteilchen, die ausschließlich oder zumindest überwiegend mindestens ein inertes Hartmaterial aufweisen, vorzugsweise eine Verbindung aus der Gruppe enthaltend Oxide, insbesondere Al₂O₃, SiO₂, ZrO₂, Karbide, insbesondere TiC, SiC, B₄C, Be₂C, Oxikarbide, Nitride, insbesondere TiN, c-BN, Si₃N₄, AlN und TiAlN, Carbonitride, natürlicher oder synthetischer Diamant sowie Bor, und in einem zweiten, sich an den ersten Schritt anschließenden Schritt eine tribochemische Behandlung mittels Strahlteilchen, die zumindest teilweise mindestens ein Salz, vorzugsweise eine Verbindung aus der Gruppe enthaltend NaCl, CaCl₂, MgCl₂, insbesondere trocken gelagertes MgCl₂, aufweisen.

Die tribochemische Behandlung ist eine mechanische und ggf. chemische Oberflächenbehandlung mittels Beschuss bzw. Bestrahlen des behandelten Teils der Körperoberfläche durch Strahlteilchen (Partikel) oder mittels Behandlung durch Mahl- oder Schleifteilchen in Gleitschleiftrommeln oder Kugelmühlen. Das Verfahren wird auch als Partikelstrahlen bezeichnet. Die Strahl-, Mahl- oder Schleifteilchen sind dabei Teilchen bestehend aus einem oder mehreren Materialien mit einer mittleren Partikelgröße im Nano- oder Mikrometerbereich. Hierbei wird die Partikelgröße unter Anwendung eines Rasterelektronenmikroskops (bei Partikelgrößen kleiner oder gleich 3 µm) oder eines Lichtmikroskops (bei Partikelgrößen größer als 3 µm) ermittelt. Die elektronen- oder lichtmikroskopischen Aufnahmen werden vermessen und aus den Messwerten, beispielsweise durch Mittelwertbildung (arithmetisches Mittel), die mittlere Partikelgröße bestimmt. Die Strahlteilchen bestehen demnach nicht aus einzelnen Atomen, Ionen oder Verbindungen sondern jeweils aus einer Gruppe oder einem Gefüge von Atomen, Ionen- oder Molekülverbindungen. Die tribochemische Behandlung wird in der Regel bei Raumtemperatur, d.h. im kalten Zustand durchgeführt. Im Folgenden wird hauptsächlich auf das Bestrahlen mittels Strahlteilchen als tribochemische Behandlungsmethode Bezug genommen. Die Ausführungen dazu gelten jedoch analog auch für die Mahl- und Schleifprozesse in Gleitschleiftrommeln und Kugelmühlen.

Die tribochemische Behandlung mindestens eines Teils der Körperoberfläche bewirkt besonders einfach und kostengünstig mittels der Strahlteilchen die Erzeugung einer Oberflächenrauhigkeit und/oder eine Beschichtung jeweils auf dem behandelten Teil der Körperoberfläche. Dabei wird die ursprünglich vorhandene Oberfläche hinsichtlich ihrer chemischen Zusammensetzung, des Verfestigungsgrades und/oder der Oberflächenmorphologie derart zielgerichtet verändert, dass eine Veränderung des Degradationsverhaltens des behandelten Implantats bewirkt wird. Die bei der tribochemischen Behandlung entstehenden Veränderungen der Oberfläche hängen insbesondere ab von der Art, der Größe, der Form und der chemischen Zusammensetzung der Strahlteilchen, von dem Strahldruck und dem Abstand zwischen der Strahldüse und dem Implantatkörper sowie dem Material des Implantatkörpers. Die tribochemische Behandlung der Körperoberfläche beseitigt ferner Oberflächenkontaminationen und bewirkt eine Kantenverrundung. Einzelheiten zu den mechanischen und/oder chemischen Veränderungen der Oberfläche und dem sich daraus ergebenden Besonderheiten des Degradationsverhaltens werden im Zusammenhang mit den nachfolgend diskutierten Ausführungsbeispielen des erfindungsgemäßen Verfahrens angegeben.

Insgesamt steht bei der tribochemischen Oberflächenbehandlung eine große Anzahl von - die späteren Oberflächeneigenschaften bestimmenden - Verfahrensparametern zur Verfügung. Diese betreffen sowohl die Eigenschaften des Strahlgutes als auch die Strahlparameter sowie optional durchzuführende Nachbehandlungsverfahren.

Ein weiterer Vorteil des erfindungsgemäßen Verfahrens besteht darin, dass bei einer tribochemischen Behandlung des Implantats das sonst bei der Herstellung von Implantaten häufig angewendete nachträgliche Elektropolieren nicht notwendig ist und insbesondere zwangsläufig entfallen muss. Durch das nachträgliche Elektropolieren würde ein Teil der aufgetragenen Beschichtung beseitigt und/oder die durch die tribochemische Behandlung verursachte Oberflächenmorphologie und somit das Degradationsverhalten verändert werden. Optional kann ein Elektropolieren vor der tribochemischen Behandlung des Implantats durchgeführt werden, um eine zusätzliche Kantenverrundung des Implantats zu erhalten.

Bei der vorliegenden Erfindung umfasst der Körper des Implantats mindestens einen Teil des Implantats, vorzugsweise den Hauptteil des Implantats, welcher die mechanische Integrität des Implantats bewirkt.

In einem bevorzugten Ausführungsbeispiels weisen die in einem ersten Schritt für die tribochemische Behandlung genutzten Strahlteilchen mindestens ein inertes Hartmaterial auf, vorzugsweise eine Verbindung aus der Gruppe enthaltend Oxide, insbesondere Al₂O₃, SiO₂, ZrO₂, Karbide, insbesondere TiC, SiC, B₄C, Be₂C, Oxikarbide, Nitride, insbesondere TiN, kubisches Bornitrid (c-BN, β-BN, "Borazon"), Si₃N₄, AlN und TiAlN, Karbonitride, natürlicher oder synthetischer Diamant sowie Bor. Diese Materialien weisen eine ausreichende Härte auf, um mit ihnen mechanische Veränderungen in der Oberfläche des Implantats derart zu erreichen, dass eine Beschleunigung der Degradation bewirkt wird. Die mechanischen Veränderungen in der Oberfläche des tribochemischen behandelten Teils des Implantats bzw. in den oberflächennahen Volumina werden nachfolgend beschrieben.

In einem weiteren Ausführungsbeispiel kann das in einem ersten Schritt verwendete Strahlteilchen elektrisch leitendes Material aufweisen. Als elektrisch leitendes (leitfähiges) Material kann beispielsweise eine Verbindung oder ein Element der Gruppe TiN, TiC, TiCN, metallische Elemente und deren Legierungen verwendet werden. Diese Strahlteilchen rufen, wenn Sie auf der Oberfläche des Implantatkörpers haften oder in ein oberflächennahes Volumen des Implantatkörpers eindringen, zusätzlich einen auf einer Lokalelementbildung beruhenden Korrosionseffekt hervor. In einem besonders bevorzugten Ausführungsbeispiel kann hartes Granulat aus einer CuZn- Legierung eingesetzt werden. Ein Teil der bei der Behandlung verwendeten CuZn- Strahlteichen adhäriert an der Oberfläche und wirkt später besonders korrosionsbeschleunigend.

Ein weiterer Vorteil der Verwendung von elektrisch leitenden Partikeln ist folgender: Es besteht die Möglichkeit, die elektrisch leitenden (leitfähigen) Strahlteilchen in einer Kammerdüse elektrisch aufzuladen und das Implantat entsprechend zur Kammerdüse entgegengesetzt zu polen (z.B. durch Anlegen einer sinusförmigen Wechselspannung). In dem so entstehenden elektrischen Feld werden die elektrisch geladenen Strahlteilchen zum Gegenpol (Implantatkörper) beschleunigt und treffen dort mit einer bestimmten, hohen kinetischen Energie auf. Diese kann durch die Parameter des elektrischen Wechselfelds (Potentialhöhe, Frequenz) variiert werden. Durch die Eigenschaften des elektrischen Wechselfelds kann der mechanische "impact", d.h. die auf die Körperoberfläche übertragene kinetische Energie und die hieraus entstehenden mechanischen Veränderungen der Körperoberfläche bzw. der oberflächennahen Volumenreiche des Körpers, zusätzlich zu den Parametern Größe und der Form (scharfkantig oder abgerundet) der Strahlteilchen gesteuert werden.

Grundsätzlich können für die oben vorgeschlagene tribochemische Behandlung auch ausschließlich elektrisch leitfähige Strahlteilchen aus metallischem Material oder elektrisch leitfähigen Hartstoffen (bspw. TiN) zusammen mit Metall-Strahlteilchen verwendet werden. Ferner besteht die Möglichkeit, eine Kombination von elektrisch leitfähigen und elektrisch nicht leitfähigen Partikeln in einer modifizierten tribochemischen Behandlungsanlage einzusetzen.

Beim Einsatz von extrem harten Strahlteilchen, die Oxide, Karbide, Nitride oder Oxikarbide enthalten, von Strahlteilchen aus elektrisch leitfähigen Materialien, die mit hoher kinetischer Energie auf den Implantatkörper auftreffen, sowie Strahlteilchen aus weiteren oben angegebenen Materialien oder aus Mischungen dieser bestehen, kommt es durch die tribochemische Behandlung zu einer Aufrauhung der Oberfläche des Implantatkörpers und zu mechanischen Oberflächenverfestigungseffekten im oberflächennahen Gefügebereich des Implantatkörpers durch lokale Kaltverformung. Diese Veränderungen der Oberfläche bewirken eine Erhöhung der kristallografischen Störstellendichte, welche eine Verringerung der Korrosionsbeständigkeit der betroffenen Bereiche des Implantatkörpers verursacht.

Durch die mechanische Einwirkung der inerten Hartpartikel (ggf. einschließlich der elektrisch leitfähigen Strahlteilchen, wenn diese eine vergleichbare Härte aufweisen) auf die Oberfläche des Implantatkörpers entstehen vorzugsweise etwa 2 µm bis etwa 5 µm tiefe und etwa 2 µm bis etwa 5 µm breite Kanäle, die meist parallel zur Einschussrichtung, aber auch schräg zu dieser verlaufen können. Diese Oberflächenstruktur wird im Folgenden auch als zerklüftete Struktur bezeichnet.

Die durch die erfindungsgemäße tribochemische Behandlung entstehende Oberflächenstruktur kann zusammenfassend mittels folgenden morphometrischen und chemischen Eigenschaften charakterisiert werden:
- Mikroaufrauhung im Rauheitsbereich Rz von mehreren µm,
- Submikroaufrauhung (Kavernen) im Rauheitsbereich Rz von < 1 µm,
- chemische Modifizierung durch adhärierte leitfähige Strahlpartikel und/oder Salze und
- eine im Vergleich zum Grundwerkstoff erhöhte Versetzungsdichte bis in eine Tiefe des Implantatkörpervolumens von bis zu etwa 5 µm ausgehend von der Körperoberfläche.

Die Mikroaufrauhung entsteht in erster Linie durch den primären Beschuss mit relativ groben Hartstoffpartikeln hoher kinetischer Energie. Diese kann sowohl durch die erhöhte Masse der Strahlpartikel als auch durch größeren Strahldruck erzielt werden. Des Weiteren kann bei Verwendung von elektrisch leitendem Material für die Strahlteilchen die kinetische Energie auch durch die elektrischen Parameter des oben beschriebenen elektrischen Wechselfeldes variiert werden. Die Submikroaufrauhung wird durch den unten beschriebenen Einsatz kleinerer Salzpartikel erzeugt, die an der Oberfläche des Implantatkörpers adhärieren und unter feuchten Umgebungsverhältnissen sofort einen Korrosionsvorgang initiieren.

Außerdem kann die zerklüftete Struktur der Körperoberfläche als Stoffreservoir für eine unten näher beschriebene weitere Beschichtung mittels einer pharmazeutisch aktiven Substanz dienen, die als Nano- oder Mikropartikel eingelagert wird und beispielsweise das Knochenwachstum fördernde Substanzen wie Kalziumphosphate, temporär wirkende Kontrastmittel und/oder zellwachstumshemmende (ggf. auch radioaktive) Substanzen umfassen kann. Ferner kann in die zerklüftete Struktur effektiv Gleitmittel zur Verringerung des Reibungskoeffizienten in einem Katheter eingelagert werden.

Insbesondere eine Verringerung der Degradationsdauer wird im erfindungsgemäßen Verfahren durch eine zweite, sich an den ersten Schritt anschließende tribochemische Behandlung erzielt, wobei die für die tribochemische Behandlung genutzten Strahlteilchen mindestens ein Salz aufweisen, vorzugsweise eine Verbindung aus der Gruppe enthaltend NaCl, CaCl₂, MgCl₂, insbesondere trocken gelagertes MgCl₂.

Die Strahlteilchen mit mindestens einem Salz werden bei einem bestimmten, vorgegebenen Druck auf die Oberfläche des Implantats gestrahlt. Der in vorteilhafter Weise anzuwendende Druckbereich liegt dabei zwischen 0,5 und 5,0 bar. Die Behandlungsdauer liegt im Bereich von 0,5 bis 30 min. Die Aufbringung eines Druckes bei der Bestrahlung bewirkt neben der Aufrauung der Körperoberfläche auch ein teilweises Adhärieren der aufgestrahlten Salze und ggf. weiterer Strahlteilchen auf der Körperoberfläche. Dabei können die Strahlteilchen, welche die korrosiv wirkenden Salze aufweisen, auch mit Strahlteilchen aus inertem Hartmaterial vermischt werden. Die auf der Oberfläche des Implantatkörpers adhärierten Salze bewirken eine beschleunigte Korrosion der Oberfläche, wobei die Korrosion auch abhängig von der anschließend an die tribochemische Behandlung durchgeführte Nachbehandlung des Implantats ist.

Insbesondere wird trocken gelagertes MgCl₂ als körniges Salz definierter Körnung als Strahlmittel ohne Zusatz von weiteren abrasiv wirkenden Hartstoffen verwendet. Trocken gelagertes MgCl₂ sollte aus dem Grund verwendet werden, da sonst Verklumpungsgefahr durch starke Hygroskopie des Materials besteht.

MgCl₂-Teilchen bewirken aufgrund ihrer geringen Härte keine wesentliche mechanische Oberflächenveränderung, d.h. keine wesentliche Oberflächenaufrauhung. Allerdings adhäriert ein großer Anteil der MgCl₂-Teilchen an der Oberfläche des Implantats. Bei anschließender Lagerung des so behandelten Implantats unter extrem trockener Atmosphäre und unter Luftabschluss wird eine Korrosion der Oberfläche zunächst verzögert. Erst zum Zeitpunkt der Implantation und dem Kontakt des Implantats, beispielsweise eines Stents, mit der Körperflüssigkeit beginnt das anhaftende MgCl₂ stark korrosiv zu wirken, so dass eine beschleunigte Degradation erfolgt.

Bei nicht ausschließlich trockener Lagerung verbleiben die MgCl₂-Partikel temporär bis zu ihrer Auflösung in der tribochemisch erzeugten Submikroaufrauhung, beschleunigen die Korrosion bei Anwesenheit von Luftfeuchtigkeit und vergrößern somit die Abmessungen der Kavität der Submikroaufrauhung.

In einem weiteren Ausführungsbeispiel weisen die im zweiten Schritt für die tribochemische Behandlung genutzten Strahlteilchen mindestens eine Verbindung aus der Gruppe enthaltend Mg, MgO und Mg(OH)₂ auf, wobei vorzugsweise der Gehalt von elementarem Magnesium am Gesamtgewicht der bei einer Behandlung eines Implantats aufgestrahlten Strahlteilchen vergleichsweise niedrig ist, insbesondere maximal 20 Gew.-% beträgt. Der restliche Teil der Strahlteilchen kann aus MgO und/oder Mg(OH)₂ bestehen. Natürlich ist zu beachten, dass bei der Verwendung von pulverförmigem Mg aus Brandschutzgründen unter Schutzgas zu arbeiten ist.

Die Behandlung mit Strahlteilchen der Zusammensetzung dieses Ausführungsbeispiels, vorzugsweise mit einer Teilchengröße (Partikelgröße) im Nanometer- bis Mikrometerbereich, führt zu einer gleichmäßigen Belegung der Körperoberfläche mit dem genannten Strahlpartikel-Material. Die so erhaltene Oberflächenbelegung mit Mg, MgO und/oder Mg(OH)₂ weist eine Schichtdicke von einigen Nanometern bis hin zu etwa 2 µm auf, welche am Implantationsort in Kontakt mit Körperflüssigkeit zu gezielt einstellbaren biochemischen Reaktionen führt. Die Beschichtung weist vorzugsweise einen Gradienten in ihrer Zusammensetzung auf, wobei der Gehalt an aufgestrahltem Mg, MgO und/oder Mg(OH)₂ in Richtung der Oberfläche des behandelten Implantats zunimmt. Die angesprochenen biochemischen Reaktionen sind beispielsweise die Verringerung der Neointimabildung bei Implantaten oder die lokale Erhöhung des pH-Wertes bei der Umwandlung von MgO zu Mg(OH)₂, die sich beispielsweise vorteilhaft bei der Medikamentenfreisetzung aus einer polymeren Deckschicht auswirkt. Insbesondere bewirkt die Anwesenheit von Magnesium auf der Oberfläche des metallischen Implantats, insbesondere wenn dieses Eisen enthält, eine Lokalelementbildung, die zu einer Aktivierung und damit einer Beschleunigung der Korrosion in dem behandelten Bereich führt.

In einem weiteren bevorzugten Ausführungsbeispiel erfolgt die tribochemische Behandlung in mindestens zwei Schritten, nämlich in einem ersten Schritt eine tribochemische Behandlung mittels Strahlteilchen, die ausschließlich oder zumindest überwiegend das mindestens eine inerte Hartmaterial aufweisen, und in einem zweiten, sich an den ersten Schritt anschließenden Schritt eine tribochemischen Behandlung mittels Strahlteilchen, die zumindest teilweise das mindestens eine Salz und/oder die mindestens eine Verbindung aus der Gruppe enthaltend Mg, MgO und Mg(OH)₂ und/oder mindestens ein metallisches Material, jeweils ggf. als Reagenz in einer Mikroverkapselung, aufweisen.

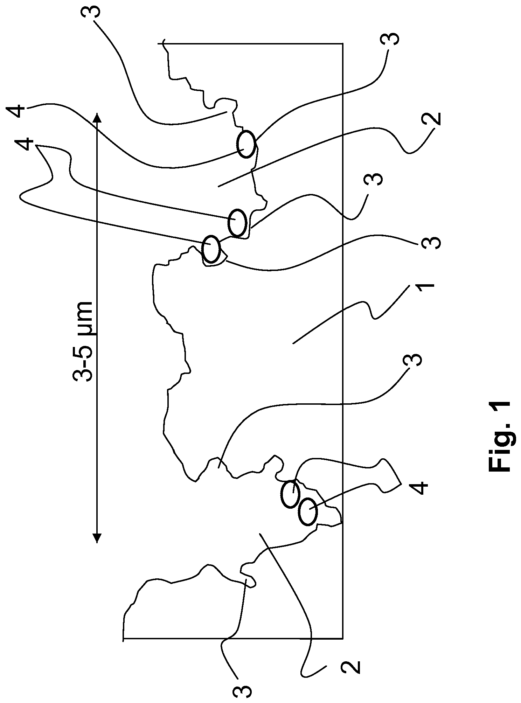

Die Durchführung des tribochemischen Verfahrens in zwei Schritten ergibt den Vorteil, dass die im zweiten Schritt aufgebrachten Strahlteilchen aufgrund der Rauhigkeit der Oberfläche, die durch den ersten Verfahrensschritt erzeugt wurde, besser haften. Ferner kann aufgrund der durch die Oberflächenzerklüftung erzielten größeren Oberfläche im zweiten Schritt eine große Gesamtmenge an Strahlteilchen mit einem insgesamt größeren Gewicht der Strahlteilchen als bei einem Verfahren ohne den ersten Schritt abgeschieden werden. Vorzugsweise haben die im zweiten Schritt aufgestrahlten Teilchen einen kleineren Durchmesser, als die im ersten Schritt aufgestrahlten Teilchen. Dies hat den Vorteil, dass die im zweiten Schritt verwendeten Teilchen tief in die im ersten Schritt erzeugte Oberflächenstruktur eindringen können (siehe auch Figur 1).

Das mindestens zweischrittige Verfahren ist auch deshalb von Vorteil, weil bei einer unten beschriebenen weiteren Beschichtung der Oberfläche mittels einer pharmazeutisch aktiven Substanz eine starke Zerklüftung der Oberfläche, die durch den ersten Verfahrensschritt erzielt wird, ebenfalls vorteilhaft ist. Der durch die Zerklüftung stark erhöhte Oberflächeninhalt, der bis zu fünf mal größer als der geometrisch aufgrund der Implantatabmessungen ermittelbare Oberflächeninhalt werden kann, bewirkt eine deutliche Erhöhung der Kapazität für die Speicherung pharmazeutisch aktiver Substanzen und somit eine Verlängerung des Zeitraums, über den diese freigesetzt werden können.

In einem weiteren Ausführungsbeispiel weisen die für die tribochemische Behandlung genutzten Strahlteilchen mindestens eine Phosphatverbindung, vorzugsweise eine Zinkphosphatverbindung, auf.

Bei diesem Ausführungsbeispiel wird eine Verlängerung der Degradation, d.h. eine Standzeitverlängerung des Implantats insbesondere bei der Verwendung eines Gemisches aus Titanoxidteilchen (z.B. P25 der Fa. Degussa) und Zinkphosphatteilchen unterschiedlicher Körnung, erreicht. Abhängig von den Verfahrensparametern wird eine mehrere 10 Nanometer dicke Schicht aus Strahlteilchen auf dem Implantatkörper ausgebildet. Selbst bei Adhäsion lediglich eines geringen Anteils dieser Partikel auf dem Material des Implantatkörpers entsteht eine die Korrosion hemmende Wirkung, die zu einer Standzeitverlängerung des Implantats unter den Bedingungen des Körpermilieus führt. Es ist hierbei von Vorteil, dass Zinkphosphat im genannten geringen Schichtdickenbereich und Titanoxid schichtdickenunabhängig keinerlei negative biochemische Reaktionen hervorrufen.

Dieses Ausführungsbeispiel des erfindungsgemäßen Verfahrens ist vor allem für orthopädische Implantate (Endoprothesen) interessant und schließt die bisher bestehende Lücke zwischen einem ("normal") über viele Monate degradierbaren Implantat und den nicht degradierbaren Implantaten aus Werkstoffen wie 316L, CoCr, Titanlegierungen usw..

In einem weiteren sehr vorteilhaften Ausführungsbeispiel weist zumindest ein Teil der Strahlteilchen ein mikroverkapseltes Reagenz auf, wobei die Mikroverkapselung vorzugsweise mindestens eine thermo- und/oder fotolabile und/oder mechanisch labile Sollbruchstelle hat.

Dieses Ausführungsbeispiel beruht auf der Verwendung mikroverkapselter Strahlteilchen, die in Richtung der Oberfläche des Implantats beschleunigt werden, wobei die Beschleunigung in Richtung der Implantatoberfläche unter unterschiedlichen Drücken, variablen Winkeln und Abständen erfolgt. Die thermo- und/oder fotolabile Wand der Mikroverkapselungen sind vorzugsweise mittels Azofunktionen (-N=N-) oder Dioxyfunktionen (-O-O-) ausgebildet.

Ferner können mechanische Sollbruchstellen in der Mikroverkapselung auf Grund von Wanddickenunterschieden vorgesehen sein, die durch eine entsprechende geometrische Oberflächenstruktur der Mikroverkapselung realisiert sind. Hierbei stellen die dünnsten Bereiche der Mikroverkapselung die mechanischen Sollbruchstellen dar, welche beim Auftreffen der mikroverkapselten Strahlteilchen auf die Implantatoberfläche brechen und die gewünschte Freisetzung des innerhalb der Mikroverkapselung angeordneten chemisch aktiven Reagenz bewirken. Dabei wird beim Auftreffen der mikroverkapselten Strahlteilchen auf die Oberfläche des Implantats zunächst die Mikroverkapselung, welche bspw. aus einem Polymermaterial besteht, massiv plastisch verformt. Der überwiegende Teil der Mikroverkapselung wird hierbei zerstört, die Hülle platzt in den Bereichen mit der geringsten Wanddicke auf und das im Inneren angeordnete chemische Reagenz wird freigelegt. Der nicht sofort von der Implantatoberfläche abprallende bzw. reflektierte Teil der mikroverkapselten Strahlteilchen, d. h. Teile der Mikroverkapselung (Hülle) und/oder mindestens ein Teil des im Inneren enthaltenen Reagenz, adhäriert an der Oberfläche des Implantats. Hierdurch wird eine chemische Reaktion in Gang gesetzt, an der das Material des Implantats, das Material der Mikroverkapselung und das freigelegten chemischen Reagenz beigefügt sind. Eine erhöhte Luftfeuchtigkeit und eine erhöhte Temperatur in der Behandlungskammer beschleunigen den durch die chemische Reaktion initiierten Korrosionsvorgang.

Als in der Mikroverkapselung enthaltenes Reagenz kann im Prinzip jede der oben angegebenen Strahlteilchen-Materialien verwendet werden. Bevorzugt kann alternativ oder zusätzlich ein bioabbaubares Polymer als Material der Mikroverkapselung und ein aktives Reagenz verwendet werden, welche zu Degradationseffekten an der Implantatoberfläche führt, die bei Einwirkung der einzelnen Komponenten so nicht zustande gekommen wären. So bewirkt bspw. eine Reaktion zwischen einem Polymer wie PLA als Material der Mikroverkapselung und NaCl, MgCl₂ oder Eisen(III)-chlorid-Hexahydrat oder Mangan(II)-chlorid als Reagenz eine starke Verschiebung des pH-Wertes an der Oberfläche des Implantats in den sauren Bereich. Die hierdurch vor allem im feuchten Milieu einsetzende heftige Korrosion führt zu einer Bildung von Eisenoxyhydroxiden. Diese haften nur schwach am Material des Implantats und werden im Laufe der fortschreitenden Korrosion durch die Chloridionen enthaltende Körperflüssigkeit (Plasma, Blut) unterwandert. Hierdurch wird eine starke Aufrauhung der Oberfläche des Implantats bewirkt, so dass sich der Oberflächeninhalt des Implantats vergrößert und die Korrosion in die Tiefe fortschreitet. Diese Effekte stellen sich bereits nach sehr kurzen Zeiträumen von wenigen Sekunden bis Minuten ein und können die durch die während der laufenden tribochemischen Oberflächenbehandlung neu herangeführten Strahlteilchen erzeugte Korrosion des Implantatmaterials noch weiter verstärken. Insgesamt entsteht somit nach der tribochemischen Oberflächenbehandlung mittels mikroverkapselten Strahlteilchen eine aufgeraute Oberfläche, die sich aufgrund Ihrer Rauheit und Oberflächenaktivität für eine weitere Oberflächenbehandlung (z. B. finales Tauchen in biodegradierbares Polymer) sehr gut eignet.

Im Rahmen der Verwendung mikroverkapselter Strahlteilchen ist es weiter bevorzugt, durch chemische Modifizierung der Mikroverkapselung und des Reagenz weitere Verbindungen auf der Oberfläche des Implantats zu erzeugen. Im Fall der Verwendung von Phosphorverbindungen (vorzugsweise Di-, Poly- oder Cyclophosphate) als Reagenz können Eisenphosphate auf der Implantatoberfläche erzeugt werden. Auf diesem Wege lassen sich in Abhängigkeit von der konkreten Zusammensetzung der Implantatoberfläche und der darin enthaltenen Anteile von Eisenoxihydroxiden und Eisenphosphaten maßgeschneiderte Degradationszeiten einstellen. Diese liegen zwischen etwa sechs und zwölf Monaten.

Weiterhin können insbesondere Metalle, deren Verbindungen oder Legierungen eingekapselt, d. h. als mikroverkapselte Reagenzien verwendet werden. So können Metalle, welche sich stark von ihrer Position in der elektrochemischen Spannungsreihe von der des Materials des Implantats unterscheiden, beim Auftreffen auf die Implantatoberfläche adhärieren. An den Stellen, an denen die Metalle adhärieren, ist die Bildung von Lokalelementen, welche die Korrosion beschleunigen, die Folge. Dies wird durch eine hohe Luftfeuchtigkeit der Umgebung begünstigt.

In einem weiteren Ausführungsbeispiel können polymerumhülltes Zinkphosphat (tertiäres Zinkeisen(II)-Phosphat), Zn₂Fe(PO₄)₂*4H₂O und/oder Hopeitkristalle (tertiäres Zinkphosphat), Zn₃(PO₄)₂*4H₂O nach einem Korrosionsprozess auf die Oberfläche des Implantats, insbesondere nach der Verwendung von mikroverkapselten Strahlteilchen, aufgebracht werden. Vorzugsweise wird zuvor die Oberfläche des Implantats getrocknet. Die Aufbringung dieser Materialien auf die Oberfläche des Implantats bewirkt - je nach den verwendeten Verfahrensparametern - einen vorläufigen Stopp des Korrosionsvorganges und ermöglicht die Langzeitlagerung des tribochemisch behandelten Implantats auch unter Nichtinertbedingungen.

In einem weiteren bevorzugten Ausführungsbeispiel erfolgt nach einer tribochemischen Behandlung eine Auslagerung des tribochemisch behandelten Implantatkörpers bei erhöhter Luftfeuchtigkeit, insbesondere bei einer Luftfeuchtigkeit größer als 80%, und/oder bei erhöhter Temperatur, insbesondere bei einer Temperatur von mehr als 50°C.

Durch die angegebene Nachbehandlung des Implantatkörpers, insbesondere nach einer tribochemischen Behandlung durch Beschuss mit Strahlteilchen, die das mindestens eine Salz enthalten, wird eine besonders beschleunigte Korrosion der Körperoberfläche erreicht. Diese Nachbehandlung, insbesondere die Auslagerung bei erhöhter Luftfeuchtigkeit, bewirkt die Entstehung von ganz spezifischen, lokal qualitativ unterschiedlichen Lochkorrosionseffekten.

Bei diesem Ausführungsbeispiel des erfindungsgemäßen Verfahrens wird das Implantat außerdem bevorzugt nach einer variablen, jedoch vorgegebenen Einwirkzeit aus der Kammer mit erhöhter Luftfeuchtigkeit und/oder erhöhter Temperatur (beispielsweise nach 24 Stunden bis 72 Stunden bei einer relativen Luftfeuchtigkeit von 80% bis 98% bei einer Temperatur von etwa 20°C bis 90°C) entnommen und ggf. mit destilliertem Wasser intensiv gespült. Zur Vermeidung von weiteren, dann unkontrolliert ablaufenden Korrosionsprozessen wird das Implantat anschließend mit einem üblichen Korrosionsinhibitor, beispielsweise Magnesiumstearat und/oder Parylene (siehe unten), versehen und trocken gelagert. Alternativ kann das behandelte Implantat auch unter Schutzgas gelagert werden. Auf den Korrosionsinhibitor kann bei einer Lagerung unter Schutzgas gegebenenfalls auch verzichtet werden.

Es ist weiterhin von Vorteil, wenn nach der tribochemischen Behandlung und ggf. nach einer Auslagerung des tribochemisch behandelten Implantatkörpers bei erhöhter Luftfeuchtigkeit und/oder erhöhter Temperatur eine weitere Beschichtung des tribochemisch behandelten Implantatkörpers zumindest auf einem Teil seiner tribochemisch behandelten Oberfläche mit Magnesiumstearat und/oder Parylene und/oder einer pharmazeutisch aktiven Substanz erfolgt.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel oder Sirolimus, bestehen.

Eine Beschichtung der tribochemisch behandelten Oberfläche des Implantats mittels Parylene und/oder Magnesiumstearat ist von Vorteil, da die Oberflächeneigenschaften, beispielsweise der während des Nachbehandlungsschritts bereits erreichte Korrosionsfortschritt, durch die darüber liegende Beschichtung 'eingefroren' werden. Hierdurch können die Oberflächeneigenschaften, die sonst ggf. von der Lagerungs- oder Transportdauer des Implantats bis zum Einbringen in den zu behandelnden Organismus abhängen, und damit auch die Degradationsdauer reproduzierbar und definiert eingestellt werden. Dieser Effekt beruht auf der Wirkung als Diffusionsbarriere gegenüber der Permeation von Wassermolekülen und Chloridionen.

Vorteilhaft wirkt sich bei einer Beschichtung mit Parylene die hohe Spaltgängigkeit von Parylene aus, so dass auch eine tiefgehende Penetration der durch die tribochemische Behandlung erzeugten Zerklüftung bis hin zu den Gründen der Zerklüftung erfolgt. Die für Parylene, insbesondere Parylene N, charakteristischen Permeationseigenschaften für Wasser, chloridhaltige Lösungen und Wasserstoff sorgen in Verbindung mit der darunter liegenden tribochemisch erzeugten Oberfläche für ein besonders gut steuerbares Degradationsverhalten des Implantats. Dieses zeichnet sich auch durch einen über den Implantatquerschnitt uniformen langsamen Korrosionsfortschritt aus. Zudem leistet die Parylene-Schicht einen zusätzlichen Beitrag zur Vermeidung bzw. Behinderung des Rissfortschritts bei mechanischer Belastung und verhindert partielle Schichtablösungen.

Parylene ist die Bezeichnung für vollständig lineare, teilkristalline, unvernetzt aromatische Polymere. Die verschiedenen Polymere besitzen unterschiedliche Eigenschaften und lassen sich in vier Grundtypen einteilen, nämlich Parylene C, Parylene D, Parylene N und Parylene F. Für die weitere Beschichtung nach einer tribochemischen Behandlung wird vorzugsweise Parylene N verwendet.

Mittels des erfindungsgemäßen Verfahrens kann bei der Beschichtung mit Magnesiumstearat ein Implantat hergestellt werden, das sich durch Defektfreiheit der Körperoberfläche durch nachträgliches Versiegeln auszeichnet. Lokale Fehlstellen und/oder auf der Körperoberfläche des Implantats vorhandene Poren und Zerklüftungen werden wirksam vor dem Kontakt mit korrosiv wirkenden Körperflüssigkeiten geschützt. Die hydrophobe Oberflächeneigenschaft und der geringe Kristallwassergehalt des Magnesiumstearats, der auch durch einen vorzugsweise durchgeführten, sich an das Aufbringen der Magnesiumstearat-Beschichtung anschließenden Trocknungsschritt erzeugt wird, bewirken während der sich anschließenden Lagerung und dem Transport des Implantats eine äußerst geringe Diffusion von Wasser zum Grundwerkstoff des Implantatkörpers. Ebenso wird das Loslösen von Partikeln mit geringer Bindungsneigung von der Oberfläche des Implantat-Körpers beim Dilatieren verhindert. Diese Partikel verbleiben in der zähen, hoch flexiblen Magnesiumstearatschicht. Hierdurch ergibt sich eine erhöhte Hämo- beziehungsweise Biokompatibilität.

Aufgrund der Magnesiumstearat-Beschichtung des Implantat-Körpers wird in vorteilhafter Weise bewirkt, dass der Reibungskoeffizient des Implantats sinkt. Hieraus folgt, dass beim Verschieben beispielsweise eines Stents als Implantat in einem Katheter geringere Kräfte aufgewendet werden müssen. Zudem werden das Crimpen und die spätere Freisetzung des Implantats an dem zu behandelnden Ort vereinfacht. So bewirkt die geringe Reibung beim Crimpen, dass das Zerkratzen der beschichteten Stentoberfläche minimiert oder bestenfalls verhindert wird.

In einem bevorzugten Ausführungsbeispiel des erfindungsgemäßen Verfahrens wird die Magnesiumstearat-Beschichtung mittels Eintauchen in eine Lösung aufgebracht, wobei die Lösung Magnesiumstearat und ein Lösungsmittel, vorzugsweise Aceton und/oder Isopropanol, enthält und vorzugsweise eine Temperatur zwischen etwa 10°C und dem jeweiligen Siedepunkt des Lösungsmittel aufweist. Alternativ kann die Magnesiumstearatschicht auch derart aufgebracht werden, dass die genannte magnesiumstearathaltige Lösung auf den Körper des Implantats aufgesprüht wird (spray coating). Dabei wird das Teil in einer Kammer an einem dünnen Draht aufgehängt und mittels eines rotierenden Tellers (Chargenhalter) allseitig besprüht.

In einem bevorzugten Ausführungsbeispiel kann die Effektivität des Tauchprozesses durch Anlegen eines Drucks erhöht werden, der kleiner als der Umgebungsdruck, vorzugsweise kleiner als etwa 90% des Umgebungsdrucks ist, d.h. des Luftdrucks an dem Ort, an dem der Tauchprozess durchgeführt wird. Der hierbei eintretende Entgasungseffekt führt zu einer schnellen Füllung der filigranen Oberflächenstruktur des Implantats mit Magnesiumstearat. Nach einer Verweildauer von einigen Minuten in der Lösung, vorzugsweise von mindestens etwa 2 Minuten, wird der mit Magnesiumstearat beschichtete Implantat-Körper aus dem Tauchbad entfernt und im Trockenofen bei einer Temperatur, die größer ist als die Raumtemperatur, vorzugsweise größer als etwa 30°C, getrocknet. Hierbei ist es besonders bevorzugt, wenn die Trocknungstemperatur möglichst gering ist, d.h. zwischen etwa 40°C und etwa 70°C liegt, da hierdurch ein langsames Freisetzen/Abdampfen des mindestens einen Lösungsmittels führt, wodurch einen porenfreie Schicht, die Magnesiumstearat enthält, erzeugt wird.

Eine oben beschriebene polymere Deckschicht eines tribochemisch behandelten Implantats weist eine um den Faktor 2 höhere Zugschälfestigkeit auf als bei einem Implantat, das nicht tribochemisch behandelt ist.

In einem weiteren Ausführungsbeispiel beträgt die mittlere Teilchengröße der Strahlteilchen etwa 5 nm bis etwa 20 µm, vorzugsweise etwa 1 µm bis etwa 3 µm. Diese Teilchengröße hat sich besonders vorteilhaft bei typischen Geometrien von Implantaten, insbesondere Stents (Stegbreiten ca. 100 µm), erwiesen. Bei einer tribochemischen Behandlung mit Teilchen von maximal einigen µm treten keine plastischen Verformungen der Implantatstruktur auf. Bei robusteren orthopädischen Implantaten können Strahlteilchen mit einer Größe von bis zu 20 µm zur Anwendung kommen. Die Messung der Partikelgröße erfolgt mit den oben angegebenen Verfahren.

In einem bevorzugten Ausführungsbeispiel enthält der Körper des Implantats vorzugsweise ein degradierbares metallisches Material, vorzugsweise überwiegend Eisen, insbesondere mehr als 90 Gew.% Eisen, besonders bevorzugt mindestens 99 Gew.% Eisen, insbesondere in einer Legierung. Als weitere metallische Materialien können alternativ oder zusätzlich Mangan, Zink und/oder Wolfram eingesetzt werden.

Diese Implantate werden, da sie kostengünstig herstellbar sind, besonders gern für die Behandlung von Erkrankungen des menschlichen oder tierischen Organismus eingesetzt. Insbesondere bei eisenhaltigen Implantaten führt die tribochemische Oberflächenbehandlung, die vorzugsweise in einem Druckbereich zwischen 0,5 bar und 5 bar durchgeführt wird, zu einer verringerten Degradationsdauer. Hierdurch wird eine Lücke zwischen den degradierbaren und nicht degradierbaren Legierungen für Implantate geschlossen.

Tribochemische Behandlungen können mit bekannten Strahlanlagen einfach und kostengünstig bewerkstelligt werden. Um die tribochemische Behandlung der gesamten Oberfläche eines Implantats mit einer Öffnung oder einem umschlossenen Hohlraum, vorzugsweise eines Stents, zu gewährleisten, werden bei dem erfindungsgemäßen Verfahren Bestrahlungseinrichtungen eingesetzt, welche ein Dorn als Strahldüse verwenden. Ein solcher Dorn weist im Bereich seiner Strahldüsen einen Durchmesser auf, der deutlich geringer ist als der Durchmesser der Öffnung oder des Hohlraums des Implantats, in die der Dorn zur Bestrahlung eingeführt wird. Beispielsweise weist der Dorn einer Bestrahlungseinrichtung zur Bestrahlung der inneren Oberfläche eines Stents im Bereich seiner Düsen einen Außendurchmesser auf, der deutlich kleiner ist als der Innendurchmesser des Stents.

Alternativ kann die tribochemische Behandlung auch in elektrischen Wechselfeldern erfolgen. Dies trifft dann zu, wenn elektrisch leitfähige Partikel (Metalle oder Hartstoffpartikel wie z.B. TiN) als Strahlteilchen zum Einsatz kommen. Dabei wird der als Strahldüse fungierende Dorn und die die Endoprothese umgebende und ebenfalls als Partikelreservoir dienende gelochte Hülse an eine Wechselspannungsquelle angeschlossen. Als Gegenpol dient das Implantat. Bei dem durch Schließen des elektrischen Kontakts startenden tribochemische Prozess werden die gegenüber der Endoprothese auf Potential liegenden Partikel aus dem Dorn und der Hülse heraus in Richtung Endoprothese beschleunigt.

Durch den einstellbaren Potentialunterschied entstehen unterschiedliche Beschleunigungseffekte, die wiederum zu unterschiedlich hohen kinetischen Energien der Partikel führen. Damit ist die Rauheit der Körperoberfläche und die Tiefenwirkung hinsichtlich Erhöhung der Versetzungsdichte einstellbar.

Die obige Aufgabenstellung wird ferner gelöst durch ein Implantat erhältlich durch ein oben beschriebenes erfindungsgemäßes Verfahren. Ein derartiges Implantat weist die oben im Zusammenhang mit dem erfindungsgemäßen Herstellungsverfahren angegebenen Vorteile auf. Die durch die tribochemische Behandlung entstehenden Oberflächenmorphologien und Oberflächenzusammensetzungen sind charakteristisch für diese Behandlung und am fertig hergestellten Implantat erkennbar.

In einem bevorzugten Ausführungsbeispiel des erfindungsgemäßen Implantats weist dieses zumindest auf einem Teil der Oberfläche des Implantatkörpers oder in einer geringen Tiefe in der Oberfläche des Implantatkörpers Strahlteilchen auf.

Insbesondere nach einer tribochemischen Behandlung mit inerten Hartpartikeln weist das erfindungsgemäße Substrat eine zerklüftete Oberflächenstruktur auf, wobei die zerklüftete Struktur die oben beschriebenen Eigenschaften und Vorteile aufweist.

Ebenfalls ist bevorzugt, wenn die Oberfläche des Implantatkörpers zumindest teilweise eine Beschichtung aufweist, welche Magnesiumstearat und/oder Parylene und/oder eine pharmazeutische aktive Substanz enthält. Hierbei liegen bevorzugte Schichtdicken der Parylene-Beschichtung zwischen etwa 0,5 und etwa 2,0 µm.

Die bevorzugte Dicke der Magnesiumstearat-Beschichtung liegt bei etwa 0,5 µm bis etwa 2,0 µm, vorzugsweise etwa 0,7 µm bis etwa 1,0 µm. Die Konzentration des Magnesiumstearats in der weiteren Beschichtung liegt dabei etwa zwischen 80 Gew.% und 100 Gew.%.

Durch die tribochemische Behandlung und die anschließende weitere Beschichtung mittels Magnesiumstearat und/oder Parylene kann die Degradationszeit des Implantats in weiten Grenzen entsprechend des jeweiligen Einsatzzwecks des Implantats variiert und definiert eingestellt werden.

Das erfindungsgemäße Verfahren bzw. das erfindungsgemäße Implantat wird nachfolgend in Beispielen anhand von Figuren erläutert. Dabei bilden alle beschriebenen und/oder bildlich dargestellten Merkmale den Gegenstand der Erfindung, unabhängig von ihrer Zusammenfassung in den Ansprüchen oder deren Rückbezüge.

Es zeigen schematisch:
- Figur 1: einen Querschnitt einer aufgerauten Oberfläche eines erfindungsgemäßen Implantats nach 24 Stunden Lagerung in feuchter Atmosphäre (80% Luftfeuchtigkeit) bei 50°C und
- Figur 2: einen Querschnitt einer elektrisch unterstützten tribochemischen Beschichtungskammer.

Figur 1 zeigt einen Querschnitt durch den oberflächennahen Bereich eines tribochemisch behandelten Implantats 1. Die Oberfläche weist Strukturen in Form von Mikroaufrauhungen 2 auf, welche durch den Beschuss mit großen Hartstoff-Strahlteilchen und/oder Strahlteilchen höherer kinetischer Energie entstehen. Die Tiefe derartiger Mikroaufrauungen beträgt wenige Mikrometer. Die Größenverhältnisse sind durch den oben in der Figur 1 gezeichneten Pfeil dargestellt, welcher eine Abmessung von 3 µm bis 5 µm symbolisiert. Eine hohe kinetische Energie kann sowohl durch eine große Masse der Strahlteilchen als auch durch einen großen Strahldruck bzw. eine hohe Geschwindigkeit der Strahlteilchen erzielt werden. Ferner kann die kinetische Energie, wie oben dargestellt, durch die elektrischen Parameter des Wechselfelds variiert werden.

Die in dem Oberflächenprofil (der Oberflächenstruktur) enthaltenen Submikroaufrauungen 3, welche deutlich kleiner sind als die Mikroaufrauungen 2, entstehen durch den Einsatz kleiner salzhaltiger Strahlteilchen, welche an der Oberfläche als Salzteilchen 4 adhärieren und unter feuchten Umgebungsbedingungen sofort korrodierend wirken. Weiterhin können an der Entstehung von Submikroaufrauhungen 3 auch die Korrosion begünstigende Reaktionen, initiiert durch das Aufstrahlen mikroverkapselter Strahlteilchen mit Sollbruchstellen in der Mikroverkapselung, beteiligt sein. Die Submikroaufrauhungen haben einen Durchmesser von etwa 10 nm bis etwa 200 nm.

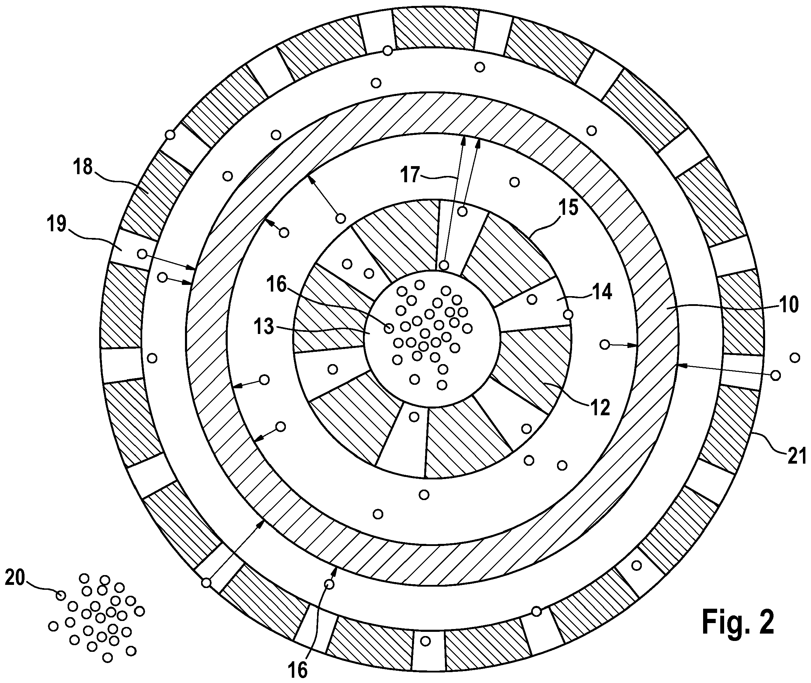

Figur 2 zeigt einen Querschnitt durch eine Anlage, mittels der die tribochemische Bestrahlung erfolgen kann. Beispielhaft wird durch die Anlage ein Stent 10 tribochemisch behandelt, welcher eine hohlzylindrische Form aufweist und somit im Querschnitt als Kreisring dargestellt ist. Im Innenvolumen des Stents 10 ist ein Dorn 12 mit einer an der Dornspitze vorgesehenen mittigen Öffnung 13 sowie radial verlaufenden Durchtrittsöffnungen 14 angeordnet. Ferner umgibt den Stent 10 eine Hülse 18 mit radial verlaufenden Durchtrittsöffnungen 19. In dem Dorn 12 und außerhalb der Hülse 18 ist ein Reservoir von Strahlteilchen 16 vorgesehen, welche von dem Dorn 12 zu Stent 10 bzw. von der Hülse 18 zu der Oberfläche des Stents 10 beschleunigt werden. Hierbei bewegen sich die Strahlteilchen 16 durch die entsprechenden Durchtrittsöffnungen 14 bzw. 19 hindurch. Die Bewegungsrichtung der Strahlteilchen 16 ist für einige Strahlteilchen durch einen Pfeil 17 angedeutet. Das Strahlteilchenreservoir der Hülse 18 ist in Figur 2 lediglich schematisch dargestellt und mit dem Bezugszeichen 20 versehen. Eine Erhöhung der kinetischen Energie der Strahlteilchen kann durch das Anlegen eines elektrischen Feldes erfolgen. Hierfür liegen einerseits der Stent 10 und anderseits der Dorn 12 bzw. die Hülse 18 auf einem unterschiedlichen elektrischen Potential. Zur Erzeugung der Potentialdifferenz weisen der Dorn 12 eine Polung 15 und die Hülse 18 eine Polung 21 auf.

### 1. Beispiel (tribochemische Behandlung eines Implantats mittels Hartstoff-Strahlteilchen)

Das 1. Beispiel betrifft eine tribochemische Behandlung eines Implantats mittels Hartpartikeln.

Die Hartpartikel bestehen aus einer Mischung aus mikroskaligen Strahlteilchen von TiC, WC und TiCN. Die mittlere Korngröße der Strahlteilchen beträgt 4 µm bei einer Streuung von +/- 1 µm. Die tribochemische Behandlung findet in einer tribochemischen Anlage statt. Mittels Druckluft bei 3 bis 4 bar Druck werden die Strahlteilchen mittels der in Figur 2 gezeigten Strahlanlage gleichzeitig auf die Innen- und die Außenseite des Implantates gestrahlt. Der Prozess erfolgt über einen Zeitraum von 5 min. Infolgedessen kommt es zu elastischen und plastischen Verformungseffekten der Oberfläche des Implantats, dessen Körper aus Reineisen oder einer Eisenbasislegierung mit Legierungsbestandteilen wie Mn, Si, Pd, Pt und/oder weiteren Bestandteilen zusammengesetzt ist. Der plastische Verformungsanteil hinterlässt die in Figur 1 dargestellte Oberflächenmorphologie (ohne die durch nachträgliche Salze entstandenen Submikroaufrauhungen).

Mikrohärteuntersuchungen eines derart behandelten Implantats aus Reineisen haben gezeigt, dass in dem durch die Strahlteilchen plastisch verformten oberflächennahen Volumenbereich eine im Vergleich zum Bauteilinneren um bis zu 150 HV 0,1 erhöhte Mikrohärte in einer Rautiefe von 10 µm vorliegt.

### 2. Beispiel (tribochemische Behandlung eines Implantats mittels Strahlteilchen aus Salzen)

Die Salze bestehen aus einer Mischung von jeweils 50 Masse% NaCl und MgCl₂. Die Strahlteilchen weisen eine Teilchengröße auf, die zwischen 200 nm und 20 µm variiert. Diese werden nach dem im Ausführungsbeispiel 1 beschriebenen Prozess auf die durch die Hartstoffpartikel bereits vorgeschädigte Oberfläche gestrahlt. Der Prozess wird in einer in Figur 2 im Schnitt dargestellten tribochemischen Behandlungsanlage bei den im Beispiel 1 genannten Drücken durchgeführt. Um ein Verklumpen der hygroskopischen Salzmischung zu vermeiden, muss diese vor und während des tribochemischen Behandlungsprozesses trocken gelagert werden.

Zusätzlich zur Beaufschlagung mit Druckluft kann die Hülse und der Dorn gegenüber dem Implantat mit einem von diesem verschiedenen elektrischen Potential beaufschlagt werden. Dies führt zu einer elektrischen Aufladung der Salzpartikel. Aus dieser resultiert eine - im Vergleich zur stromlosen Behandlungsvariante - erhöhte Beschleunigung der Partikel in Richtung Implantatoberfläche. Eine erhöhte temporäre Anhaftung der Salze in den Mikroaufrauungen und die daraus resultierende stärkere Ausprägung von Submikroaufrauhungen sind die Folge.

Die Chlorid-Strahlteilchen werden beim Auftreffen auf die Oberfläche des Implantats in viele kleine Fragmente zerlegt, welche teilweise von der Oberfläche zurückreflektiert werden und an dieser abprallen. Die Fragmente der Strahlteilchen, welche an der Oberfläche adhärieren, reagieren in der oben beschriebenen Weise mit dem Implantatmaterial und erzeugen die Submikroaufrauhungen.

### 3. Beispiel (tribochemische Behandlung eines Implantats mittels Strahlteilchen aus Mg, MgO und/oder Mg(OH)₂)

Die tribochemischen Behandlungsparameter für eine Bestrahlung mittels der in diesem Beispiel verwendeten Strahlteilchen sind mit den im Ausführungsbeispiel 1 genannten Parametern identisch. Allerdings werden auf Grund der geringeren Härte der Strahlteilchen eine geringere plastische Verformung und eine geringere Aufrauung der Oberfläche des Implantats bewirkt. Das Mischungsverhältnis der Strahlteilchen setzt sich zusammen aus 30 Masse% Mg (mittlere Partikelgröße 10 µm), 30 Masse% MgO (mittlere Partikelgröße 10 µm) und 40 Masse% Mg(OH)₂ mit einer mittleren Partikelgröße von 3,5 µm. Dieses Mischungsverhältnis verringert einerseits die Gefahr der Selbstentzündung, andererseits bewirkt die teilweise Anhaftung des metallischen Mg auf der Implantatoberfläche, welche bspw. Eisen enthält, eine starke Lokalelementbildung. Durch die Leitfähigkeit des Magnesiums ergibt sich auch hier die Möglichkeit der Teilchenbeschleunigung durch das Anlegen elektrischer Wechselfelder. Dabei werden fast ausschließlich die Mg-Strahlteilchen beschleunigt und auf die Endoprothesenoberfläche gestrahlt. Durch wechselseitigen Betrieb der tribochemischen Behandlungsanlage (mit und ohne elektrisches Wechselfeld) können so tribochemisch erzeugte Mehrlagenschichten generiert werden.

### 4. Beispiel (tribochemische Behandlung eines Implantats mittels Titanoxid oder einem Gemisch aus Titanoxid und Zinksphosphat)

Verfahrensparameter und Partikelgrößen wie bei Beispiel 1.

Für die Anwendung als Strahlteilchen im erfindungsgemäßen Verfahren ist unwesentlich, in welcher Modifikation (Rutil, Anatas oder Brookit) das Titanoxid vorliegt. Die elektrische Wechselfeldunterstützung zur Beschleunigung von Strahlteilchen kann nicht zur Anwendung gebracht werden, da die Partikel nicht leitfähig sind. Durch die geringe Härte der Strahlteilchen finden nur eine geringe Verfestigung der Oberfläche und nur eine geringe Aufrauung statt. Die adhärierten Partikel beeinflussen im Gegensatz zu den anderen Ausführungsbeispielen das Degradationsverhalten in Richtung einer verzögerten Degradation (z.B. Standzeit eines Eisenbasisstents wird auf das 1,5-fache erhöht).

### 5. Beispiel (wie eines der Beispiele 1. bis 4. und zusätzliche weitere Beschichtung mittels Parylene, Magnesiumstearat und/oder pharmazeutisch aktiver Substanz).

Auf der Basis aller bisher genannten Ausführungsbeispiele kann als finaler Behandlungsschritt eine Beschichtung mit o.g. Materialien erfolgen. Die Beschichtung mit Parylene C erfolgt aus der Gasphase. Nach ca. einer halben Stunde Beschichtungszeit wird eine Schichtdicke von ca. 0,5 µm erzielt.

Die Parylene-Beschichtung verfolgt dabei das Ziel eines temporären Korrosionsschutzes. Der "vorgeschädigte" Oberflächenzustand wird "eingefroren". Es erfolgt somit keine unkontrollierte selbstständig verlaufende Degradation vor dem Verbringen der Endoprothese an den Einsatzort.

Das gleiche Ziel wird mit der nachfolgend beschriebenen Magnesiumstearatbeschichtung verfolgt. Nach der Durchführung der Ausführungsbeispiele 1 bis 4 und einer daran anschließenden Trocknung, wird die Endoprothese auf einen Kunststofffaden (z.B. Polyamid) aufgehängt und anschließend in die Lösung zur Auftragung des Magnesiumstearats getaucht. Die Lösung besteht z.B. aus 9 Teilen hochreinem Aceton oder Isopropanol und 1 Teil Magnesiumstearat. Der Tauchvorgang erfolgt bei Raumtemperatur in einem evakuierbaren Exsikkator. In diesem wird ein Unterdruck von ca. 100 mbar mittels einer Pumpe erzeugt. Hierdurch werden die filigranen und durch die vorangegangene plasmachemische Vorbehandlung entstandenen mikroporösen Oberflächenstrukturen bzw. die Hinterschneidungen und kompliziert geformten Strukturen effektiv von Restgas befreit. Dadurch kann in der Lösung eine vollständige Bedeckung der Stentoberfläche durch das Magnesiumstearat erfolgen, das auch in die Oberflächenstrukturen und Hinterschneidungen eindringt. Nach einer Verweildauer von etwa 3 Minuten in dem Tauchbad wird der Exsikkator belüftet, das Implantat aus dem Tauchbad entnommen und anschließend in einem Umluftschrank, immer noch am Kunststofffaden hängend, bei einer Temperatur von 60°C getrocknet. Die Schichtdicke der so erhaltenen Magnesiumstearat-Beschichtung liegt dabei im Bereich von etwa 0,5 bis etwa 10 µm.

Bedingt durch den im Exsikkator vorliegenden Unterdruck wird das Magnesiumstearat sehr gleichmäßig auf der Oberfläche abgeschieden. Eine geringe Trocknungstemperatur bewirkt in vorteilhafter Weise ein langsames Freisetzen/Abdampfen der Lösungsmittel der Tauch-Lösung, so dass eine porenfreie Magnesiumstearatschicht entsteht. Handelt es sich bei dem so hergestellten Implantat um einen Stent, so kann der mit der ersten Schicht und der Zwischenschicht versehene Körper anschließend mit einem Katheter komplettiert und einer Strahlensterilisation unterzogen werden.

Analog zu der Herstellung der Parylene- oder Magnesiumstearat-Beschichtung kann die zerklüftete Oberfläche des Implantats alternativ oder zusätzlich mit einer pharmazeutisch aktiven Substanz beschichtet werden. Bevorzugte Substanzen sind oben in der Beschreibung angegeben.

### Bezugszeichenliste:

- 1: Implantatkörper
- 2: Mikroaufrauung
- 3: Submikroaufrauung
- 4: adhäriertes Salzteilchen
- 10: Stent
- 12: Dorn
- 13: Öffnung
- 14: Durchtrittsöffnung für Strahlteilchen 16
- 15: Polung des Dorns
- 16: Strahlteilchen
- 17: Pfeil (Bewegungsrichtung des jeweiligen Strahlteilchens 16)
- 18: Hülse
- 19: Durchtrittsöffnung für Strahlteilchen 16
- 20: Strahlteilchenreservoir der Hülse 18
- 21: Polung der Hülse 18

## Patentansprüche

1. Verfahren zur Herstellung eines Implantats (1, 10), insbesondere einer intraluminalen Endoprothese, mit einem Körper enthaltend metallisches Material, vorzugsweise Eisen, umfassend die folgenden Schritte:
a) Bereitstellen des Körpers des Implantats (1, 10),
b) tribochemische Behandlung mindestens eines Teils der Körperoberfläche mittels Strahlteilchen (16),
**dadurch gekennzeichnet, dass** die tribochemische Behandlung in mindestens zwei Schritten erfolgt, nämlich in einem ersten Schritt eine tribochemische Behandlung mittels Strahlteilchen, die ausschließlich oder zumindest überwiegend mindestens ein inertes Hartmaterial aufweisen, vorzugsweise eine Verbindung aus der Gruppe enthaltend Oxide, insbesondere Al₂O₃, SiO₂, ZrO₂, Karbide, insbesondere TiC, SiC, B₄C, Be₂C, Oxikarbide, Nitride, insbesondere TiN, c-BN, Si₃N₄, AlN und TiAlN, Carbonitride, natürlicher oder synthetischer Diamant sowie Bor, und in einem zweiten, sich an den ersten Schritt anschließenden Schritt eine tribochemische Behandlung mittels Strahlteilchen, die zumindest teilweise mindestens ein Salz, vorzugsweise eine Verbindung aus der Gruppe enthaltend NaCl, CaCl₂, MgCl₂, insbesondere trocken gelagertes MgCl₂, aufweisen.

2. Verfahren nach Anspruch 1, **dadurch gekennzeichnet, dass** an einer Bestrahlungseinrichtung, welche die für die tribochemische Behandlung verwendeten Strahlteilchen bereitstellt und vorzugsweise einen Dorn (12) und/oder eine Hülse (18) aufweist, ein von dem elektrischen Potential des Implantatkörpers (10) verschiedenes elektrisches Potential anliegt.

3. Verfahren nach einem der vorhergehenden Ansprüche, **dadurch gekennzeichnet, dass** die Strahlteilchen ein mikroverkapseltes Reagenz aufweisen, wobei die Mikroverkapselung vorzugsweise mindestens eine thermo- und/oder photolabile und/oder mechanisch labile Sollbruchstelle hat, wobei die Reagenz vorzugsweise mindestens eines der in Anspruch 1 angegebenen Elemente und Verbindungen der Strahlteilchen aufweist und/oder mindestens eine Verbindung der Gruppe PLA, MgCl₂, Eisen(III)-chlorid-Hexahydrat, Mangan(II)-chlorid, Diphosphate, Polyphosphate, Cyclophosphate.

4. Verfahren nach einem der vorhergehenden Ansprüche, **dadurch gekennzeichnet, dass** der zweite Schritt der tribochemischen Behandlung mittels Strahlteilchen erfolgt, die zumindest teilweise mindestens eine Verbindung aus der Gruppe enthaltend Mg, MgO und Mg(OH)₂ und/oder mindestens eine elektrisch leitende Material, ggf. jeweils als mikroverkapselte Reagenz, aufweisen.

5. Verfahren nach einem der vorhergehenden Ansprüche, **dadurch gekennzeichnet, dass** die für die tribochemische Behandlung genutzten Strahlteilchen mindestens eine Phosphatverbindung, vorzugsweise eine Zinkphosphatverbindung, aufweisen.

6. Verfahren nach einem der vorhergehenden Ansprüche, **dadurch gekennzeichnet, dass** nach einer tribochemischen Behandlung eine Auslagerung des tribochemisch behandelten Implantatkörpers bei erhöhter Luftfeuchtigkeit, insbesondere bei einer Luftfeuchtigkeit größer als 80%, und/oder bei erhöhter Temperatur, insbesondere bei einer Temperatur von mehr als 50°C, erfolgt.

7. Verfahren nach einem der vorhergehenden Ansprüche, **dadurch gekennzeichnet, dass** nach der tribochemischen Behandlung und ggf. nach einer Auslagerung des tribochemisch behandelten Implantatkörpers bei erhöhter Luftfeuchtigkeit und/oder erhöhter Temperatur eine Beschichtung des tribochemisch behandelten Implantatkörpers zumindest auf einem Teil seiner tribochemisch behandelten Oberfläche mit Magnesiumstearat und/oder Parylene und/oder einer pharmazeutisch aktiven Substanz und/oder Zinkphosphate und/oder Hopeitkristallen erfolgt.

8. Verfahren nach einem der vorhergehenden Ansprüche, **dadurch gekennzeichnet, dass** die mittlere Teilchengröße der Strahlteilchen etwa 5 nm bis etwa 20 µm beträgt.

9. Verfahren nach einem der vorhergehenden Ansprüche, **dadurch gekennzeichnet, dass** der Körper des Implantats überwiegend Eisen aufweist, vorzugsweise mehr als 90 Gew.%, besonders bevorzugt mehr als 99 Gew.% Eisen, insbesondere in einer Legierung, enthält.

10. Implantat, insbesondere intraluminale Endoprothese, mit einem Körper enthaltend metallisches Material, vorzugsweise Eisen, erhältlich durch ein Verfahren gemäß einem der vorhergehenden Ansprüche.

11. Implantat nach Anspruch 10, **dadurch gekennzeichnet, dass** die Oberfläche des Implantats eine zerklüftete Struktur aufweist.

12. Implantat nach Anspruch 10 oder 11, **dadurch gekennzeichnet, dass** zumindest auf einem Teil der Oberfläche des Implantatkörpers oder in einer geringen Tiefe dieser Oberfläche Strahlteilchen angeordnet sind.

13. Implantat nach einem der Ansprüche 10 bis 12, **dadurch gekennzeichnet, dass** die Oberfläche des Implantatkörpers zumindest teilweise eine Beschichtung aufweist, welche Magnesiumstearat und/oder Parylene und/oder eine pharmazeutisch aktive Substanz enthält.

## Claims

1. A method for producing an implant (1, 10), in particular an intraluminal endoprosthesis, having a body containing metal material, preferably iron, comprising the following steps:
a) providing the body of the implant (1, 10),
b) tribochemically treating at least part of the body surface by means of blasting particles (16),
**characterised in that** the tribochemical treatment occurs in at least two steps, specifically in a first step a tribochemical treatment by means of blasting particles which comprise exclusively or at least predominantly at least one inert hard material, preferably a compound from the group containing oxides, in particular Al₂O₃, SiO₂, ZrO₂, carbides, in particular TiC, SiC, B₄C, Be₂C, oxycarbides, nitrides, in particular TiN, c-BN, Si₃N₄, AlN and TiAIN, carbonitrides, natural or synthetic diamond, and boron, and in a second step following on from the first step a tribochemical treatment by means of blasting particles which comprise at least in part at least one salt, preferably a compound from the group containing NaCl, CaCl₂, MgCl₂, in particular dry-stored MgCl₂.

2. The method according to claim 1, **characterised in that** an electrical potential different from the electrical potential of the implant body (10) is applied to an irradiation installation which provides the blasting particles used for tribochemical treatment and preferably has a mandrel (12) and/or a sleeve (18).

3. The method according to either one of the preceding claims, **characterised in that** the blasting particles comprise a microencapsulated reagent, wherein the microencapsulation preferably has at least one thermolabile and/or photolabile and/or mechanically labile predetermined breaking point, wherein the reagent preferably comprises at least one of the elements and compounds of the blasting particles specified in claim 1 and/or at least one compound of the group PLA, MgCl₂, iron(III) chloride hexahydrate, manganese(II) chloride, diphosphates, polyphosphates, cyclophosphates.

4. The method according to any one of the preceding claims, **characterised in that** the second step of the tribochemical treatment occurs by means of blasting particles comprising, at least in part, at least one compound from the group containing Mg, MgO and Mg(OH)₂ and/or at least one electrically conductive material, in each case as a microencapsulated reagent where applicable.

5. The method according to any one of the preceding claims, **characterised in that** the blasting particles used for the tribochemical treatment comprise at least one phosphate compound, preferably a zinc phosphate compound.

6. The method according to any one of the preceding claims, **characterised in that,** following tribochemical treatment, storage of the tribochemically treated implant body is conducted at increased humidity, in particular at a humidity greater than 80%, and/or at increased temperature, in particular at a temperature of more than 50 °C.

7. The method according to any one of the preceding claims, **characterised in that,** following tribochemical treatment and, where applicable, following storage of the tibochemically treated implant body at increased humidity and/or increased temperature, the tribochemically treated surface is coated at least in part with magnesium stearate and/or parylene and/or a pharmaceutically active substance and/or zinc phosphate and/or hopeite crystals.

8. The method according to any one of the preceding claims, **characterised in that** the mean particle size of the blasting particles is approximately 5 nm to approximately 20 µm.

9. The method according to any one of the preceding claims, **characterised in that** the body of the implant predominantly comprises iron, preferably more than 90 % by weight, particularly preferably more than 99 % by weight iron, in particular in an alloy.

10. An implant, in particular an intraluminal endoprosthesis, having a body containing metal material, preferably iron, obtainable by a method according to any one of the preceding claims.

11. The implant according to claim 10, **characterised in that** the surface of the implant has a fissured structure.

12. The implant according to claim 10 or 11, **characterised in that** blasting particles are arranged at least on part of the surface of the implant body or at a shallow depth from this surface.

13. The implant according to any one of claims 10 to 12, **characterised in that** the surface of the implant body has a coating at least in part, which coating contains magnesium stearate and/or parylene and/or a pharmaceutically active substance.

## Revendications

1. Procédé de fabrication d'un implant (1, 10), notamment une endoprothèse intraluminale, avec un corps contenant un matériau métallique, de préférence du fer, comprenant les étapes suivantes :
a) mise au point du corps d'implant (1, 10),
b) traitement tribochimique d'au moins une partie de la surface du corps au moyen de particules de rayonnement (16),
**caractérisé en ce que** le traitement tribochimique s'effectue en au moins deux étapes, à savoir, dans une première étape, un traitement tribochimique au moyen de particules de rayonnement qui présentent exclusivement ou au moins majoritairement au moins un matériau dur inerte, de préférence un composé provenant du groupe contenant des oxydes, notamment Al₂O₃, SiO₂, ZrO₂, des carbures, notamment TiC, SiC, B₄C, Be₂C, des oxycarbures, des nitrures, notamment TiN, c-BN, Si₃N₄, AIN et TiAIN, des carbonitrures, du diamant naturel ou synthétique comme du bore, et dans une deuxième étape, une étape suivant la première étape, un traitement tribochimique au moyen de particules de rayonnement qui présentent au moins un sel, de préférence un composé provenant du groupe contenant NaCl, CaCl₂, MgCl₂, notamment du MgCl₂ stocké dans un lieu sec.

2. Procédé selon la revendication 1, **caractérisé en ce qu**'au niveau d'un dispositif de rayonnement, lequel met à disposition les particules de rayonnement employées pour le traitement tribochimique et présente de préférence un mandrin (12) et/ou une cosse (18), il y a un potentiel électrique différent du potentiel électrique du corps d'implant (10).

3. Procédé selon l'une des revendications précédentes, **caractérisé en ce que** les particules de rayonnement présentent un réactif micro-encapsulé, où la microencapsulation a de préférence au moins un point de rupture souhaitée thermolabile, et/ou photolabile, et/ou labile mécaniquement, où le réactif présente de préférence au moins un des éléments indiqué en revendication 1 et des composés des particules de rayonnement, et/ou au moins un composé du groupe du PLA, du MgCl₂, du chlorure de fer (III) hexahydraté, du dichlorure de manganèse (II), des diphosphates, des polyphosphates, des cyclophosphates.

4. Procédé selon l'une des revendications précédentes, **caractérisé en ce que** la deuxième étape du traitement tribochimique s'effectue au moyen de particules de rayonnement qui présentent au moins partiellement au moins un composé provenant du groupe contenant Mg, MgO, et Mg(OH)₂ et/ou au moins un matériau électriquement conducteur, éventuellement, chaque fois, sous la forme d'un réactif micro-encapsulé.

5. Procédé selon l'une des revendications précédentes, **caractérisé en ce que** pour le traitement tribochimique, des particules de rayonnement utilisées présentent au moins un composé de phosphate, de préférence un composé de phosphate de zinc.

6. Procédé selon l'une des revendications précédentes, **caractérisé en ce qu**'après un traitement tribochimique il y a un relargage du corps d'implant traité par tribochimie pour une humidité de l'air élevée, notamment pour une humidité de l'air supérieure à 80 %, et/ou pour une température élevée, notamment pour une température supérieure à 50 °C.

7. Procédé selon l'une des revendications précédentes, **caractérisé en ce qu**'après le traitement tribochimique, et éventuellement, après un relargage du corps d'implant traité par tribochimie, pour une humidité de l'air élevée, et/ou pour une température élevée, il y se produit un revêtement du corps d'implant traité par tribochimie au moins sur une partie de sa surface traitée par tribochimie avec du stéarate de magnésium, et/ou des parylènes, et/ou une substance active d'un point de vue pharmaceutique, et/ou du phosphate de zinc et/ou des cristaux d'hopéite.

8. Procédé selon l'une des revendications précédentes, **caractérisé en ce que** la taille particulaire moyenne des particules de rayonnement est d'environ 5 nm à environ 20 µm.

9. Procédé selon l'une des revendications précédentes, **caractérisé en ce que** le corps de l'implant présente principalement du fer, de préférence plus de 90 % en poids, de manière particulièrement préférée plus de 99 % en poids de fer, notamment dans un alliage.

10. Implant, notamment endoprothèse intraluminale, avec un corps contenant un matériau métallique, de préférence du fer, pouvant être obtenu par un procédé selon l'une des revendications précédentes.

11. Implant selon la revendication 10, **caractérisé en ce que** la surface de l'implant présente une structure découpée.

12. Implant selon la revendication 10, ou 11, **caractérisé en ce que** des particules de rayonnement sont disposées sur au moins une partie de la surface du corps d'implant ou à une faible profondeur de cette surface.

13. Implant selon l'une des revendications 10 à 12, **caractérisé en ce que** la surface du corps d'implant présente au moins partiellement un revêtement, lequel contient du stéarate de magnésium, et/ou des parylènes, et/ou une substance active d'un point de vue pharmaceutique.
